# EUROPEAN PATENT APPLICATION

(11) **EP 4 796 057 A1**
(43) Date of publication of application: **26.08.2026**
(21) Application number: 24847274.8
(22) Date of filing: 19.11.2024
(51) Int. Cl.: A61B 17/29

(54) **HEAD FOR A LAPAROSCOPIC SURGICAL INSTRUMENT**

(30) Priority: 27.11.2023 ES 202330981
(71) Applicant: Servocad Microtronics S.L., 08272 Barcelona (ES)
(72) Inventor: HERNANDEZ JUANPERA, Jesus, 08272 SANT FRUITOS DEL BAGES (BARCELONA) (ES)
(74) Representative: Isern Patentes y Marcas S.L.
(86) International application number: PCT/ES2024/070718
(87) International publication number: WO 2025/114624

(57) **Abstract**

Head for a laparoscopic surgical instrument, comprising two jaws (70, 71) hinged together through a pivoting shaft, and an actuator system connected with each of the jaws (70, 71) in such a way that they are capable of moving rotatively independently, the actuator system being enabled to connect to movable gripping members. The actuator system comprises an actuator set for each of the jaws (70, 71) that has a pusher member enabled to be coupled to a pushrod (5, 6), the pusher member (73) being linked to a corresponding jaw through a connecting rod member (74), in such a way that the jaw (70, 71) can rotate with respect to the connecting rod member (74) by a linear displacement of the pusher member (73), taking a tangential arrangement between a jaw edge section (70, 71) and an edge section of the pusher member (73) during the entire stroke of the jaw rotational movement (70, 71).

## Description

### OBJECT OF THE INVENTION

The object of the present invention is to provide a head for a laparoscopic surgical instrument.

More specifically, the invention proposes the development of a head for a laparoscopic surgical instrument, as well as an instrument equipped with such a head, which allows a greater degree of opening between jaws that are part of the work tool.

### BACKGROUNDS OF THE INVENTION

Nowadays, laparoscopic surgical devices are known for surgical operations that require the suturing and ligation of body tissues through incisions made in the walls of the body. However, in a large majority of these devices, the surgical tool acts on a single plane, thus limiting the degrees of freedom and consequently making it difficult for the surgeon to use.

Documents ES 2385518 and ES 1230155U are well known, disclosing laparoscopic devices that satisfactorily solve the problem previously mentioned.

However, due to the constructive configuration of the head, the degree of opening of the jaws is still limited by not allowing a rotation of up to 180°, so that these instruments have a limited scope of application or can make it difficult for the surgeon to perform a surgical operation.

In the case of the head shown in ES 1230155U, although the head has a terminal region provided with a groove to extend the degree of opening of the jaws, the configuration of the pivoting shaft prevents a higher degree of openness, so it is not possible to achieve a maximum opening at an angle of 180° between jaws.

Also, it is known US 4763669 disclosing a surgical instrument provided with a head with jaws at one end, the common characteristics of which form part of the preamble of claim 1.

Therefore, there is still a need to develop a head for laparoscopic instruments that allow the independent movement of each of the jaws that are part of the head and at the same time a degree of opening between jaws of up to 180 degrees.

Furthermore, the applicant is not currently aware of an invention that has all the characteristics described in this specification.

### DESCRIPTION OF THE INVENTION

The present invention has been developed in order to provide a head for a surgical instrument that is a novelty within the field of application and solves the aforementioned drawbacks, further contributing other additional advantages that will be evident from the description below.

It is therefore an object of the present invention to provide a head for a laparoscopic surgical instrument, comprising two movable jaws hinged together through a pivoting shaft, and an actuating system connected with each of the jaws in such a way that they are capable of moving rotationally independently, the actuator system is enabled to connect to movable gripping members.

In particular, the invention is characterized in that the actuator system comprises an actuator set for each of the jaws that has a pusher member enabled to be coupled to a pushrod, the pusher member being linked in rotary motion with a corresponding jaw through a connecting rod member, in such a way that the linear displacement of the pusher member implies a rotary movement of the connecting rod member, which in turn implies the rotary movement of the jaw. This connecting rod member also comes into contact with its linked jaw during the closing movement of the latter.

The pivoting shaft that joins the two jaws is made up of a tongue and groove system formed by two parts aligned with each other, and in which a tangential arrangement is taken between a section of the edge of the jaw and a section of the edge of the pusher member during the rotational movement of the jaw.

According to the invention, the tongue and groove system of the pivoting shaft of the two jaws is made up of a protruding portion that extends internally from one of the jaws and can be attached in a rotary manner to a recess present on the inside of the opposite jaw.

According to the invention, the connecting rod member has an oblong development provided on a longer side of a curved contour recess intended to transversely accommodate the pivoting shaft.

Advantageously, the pivoting shaft that joins the two jaws is located at a point proximal to the flange present in the rear region of the jaw, so that it allows the rotational movement of each of the jaws of 90 degrees with respect to a longitudinal axis of the head, either in an upward or downward direction during use.

In order to achieve the rotational movement of each of the jaws of 90°, a cam system is provided between the jaw and the pusher member, such system being formed by at least one protrusion present in the flange section of the pusher member and at least one protrusion in the flange section of the rear region.

In addition, guide means are provided to guide the path of the connecting rod member and the pusher member connected to a jaw during the angular movement of the jaw, so as to ensure that the member s involved in the movement of the jaw make the correct path.

Preferably, the guide means may include at least one projection protruding from a side face of the pusher member that can be moved along a slot present in a cover.

Also preferably, the guide means include at least one protrusion protruding from a side face of the connecting rod member that is movable along a slot present in a cover.

According to the invention, each actuator set comprises a cap.

Thanks to these characteristics, a head is obtained that is intended to be mounted on a laparoscopic device with manual (or even robotic) actuation and manipulation, where the surgeon will be able to have a larger work area since each of the jaws has a greater opening/closing angle, being up to 180 degrees for each of the jaws unlike other known heads where angular movement is more limited.

The fact that it has this greater angular movement allows the head to be used for a greater variety of different surgical interventions and by having a chain arrangement of solids, that is, the members that intervene in the movement are caused by a pushing action between them, it allows the size of the head to be decreased, maintaining a structural rigidity that prevents the head from breaking, thus expanding the possibilities of use of the laparoscopic device.

It is another object of the invention to provide a laparoscopic surgical instrument comprising:
- A hand-gripping region provided with two movable gripping members hinged to perform an angular and independent movement, each enabled to insert a user's finger, and a third fixed gripping member enabled to insert a user's finger, and
- An actuator mechanism with push rods that are each coupled to a corresponding movable gripping member in such a way that they transmit the angular movement of the gripping members to a head such as the one explained above.

The head for a surgical instrument described therefore represents an innovative structure with structural and constitutive characteristics hitherto unknown for the purpose for which it is intended, reasons which, together with its practical utility, provide it with sufficient grounds to obtain the privilege of exclusivity requested.

Other characteristics and advantages of the head for a surgical instrument that is the subject of the present invention will be evident from the description of a preferred, but not exclusive, embodiment, which is illustrated by way of non-exhaustive example in the accompanying drawings, in which:

### BRIEF DESCRIPTION OF THE DRAWINGS

Figure 1 is a lateral elevation view of a laparoscopic surgical instrument provided with a head in accordance with the present invention;
Figure 2 is a side elevation view of the instrument shown in figure 1, in which some parts have been omitted

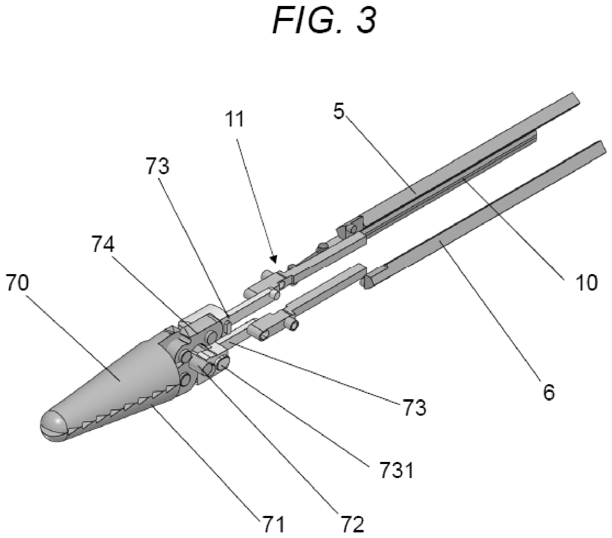

Figure 3 is a partial perspective view of the region where the work tool is located where some parts have been extracted for clarity purposes;
Figure 4 is a cross-sectional view along the A-A line shown in Figure 1;

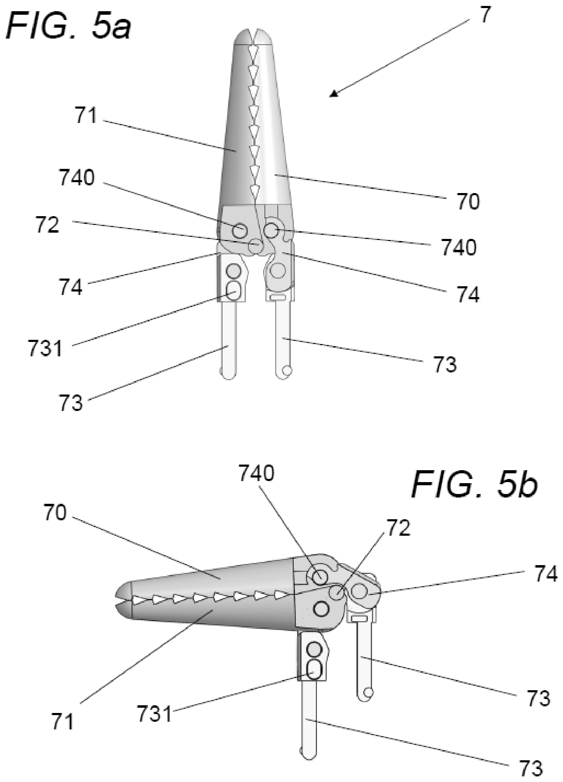

Figures 5a and 5b are lateral elevation views of the head for a surgical instrument according to the invention, in which the two jaws are facing each other in a closed position;

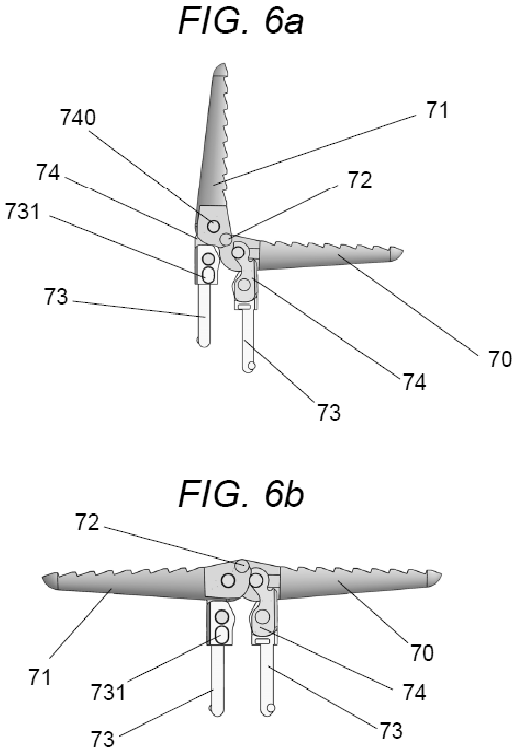

Figures 6a and 6b are lateral elevation views of the head according to the invention in which one of two jaws is in an open position while the other jaw is in a resting and completely open position, respectively;
Figures 7a-7d are a sequential lateral elevation view that shows different positions adopted by an actuator set and a connected jaw;
Figure 8 is a perspective view of two jaws that are part of the head according to the invention;

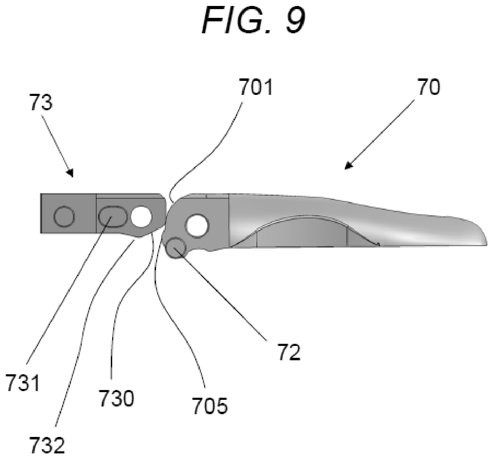

Figure 9 is a schematized elevation view where only the jaw and partially the pusher member are shown;
Figure 10 is a perspective view of a cap that is part of an actuator set of the head of the invention; and
Figure 11 is an isometric view of a connecting rod member that is part of an actuator set.

### DESCRIPTION OF A PREFERRED EMBODIMENT

In view of the aforementioned figures and, in accordance with the numbering adopted, it is the figures show a preferred exemplary embodiment of the invention, which comprises the parts and elements indicated and described in detail below.

Furthermore, the terms first, second, third and the like in the description and claims are used to distinguish between similar elements and not necessarily to describe a sequential or chronological order. The terms may be interchanged under appropriate circumstances and the embodiments of the invention may operate in sequences other than those described or illustrated herein.

In addition, the terms top, bottom, top, bottom, and the like in the description and claims are used for descriptive purposes and not necessarily to describe relative positions.

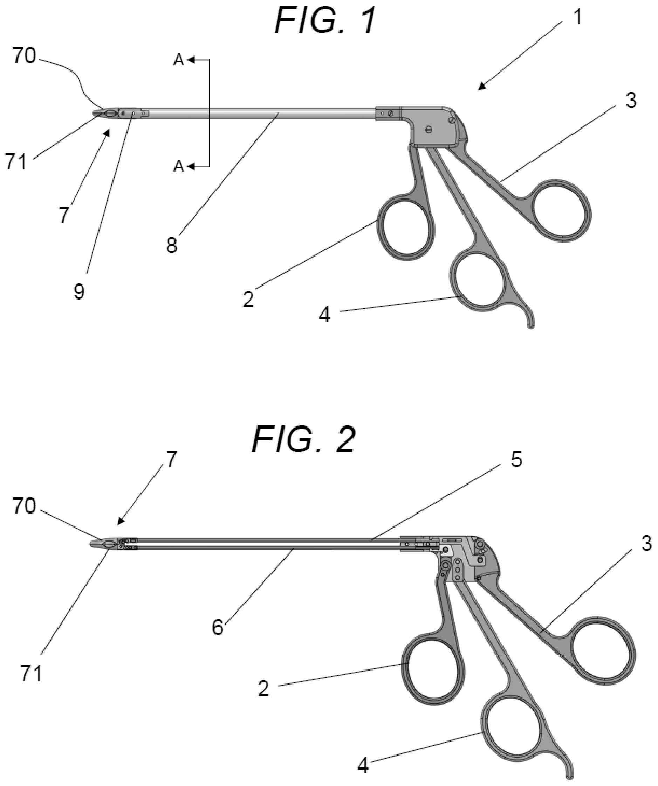

As can be seen in Figures 1 and 2, a laparoscopic surgical instrument is shown, generally indicated by reference (1), which comprises a hand-gripping region provided with two movable gripping members (2, 3) hinged to perform an angular movement each, and enabled to insert a user's finger, and a third fixed gripping member (4) configured to insert a user's finger. The three grip members (2, 3, 4) are made up of an elongated section that ends in a thimble section.

In addition, an actuating mechanism is provided with push rods (5, 6), each of which is coupled to a corresponding movable gripping members (2, 3), in such a way that they transmit the angular movement of the gripping members to a head, generally indicated by reference (7), which corresponds to the work tool equipped with two jaws (70, 71), which is described in greater detail below. Thus, the movable gripping member (2) to the left of the fixed gripping member (4) is responsible for the angular movement of the jaw (71) located at the bottom according to the figure, while the movable gripping member (3) located to the right of the fixed gripping member (4) is intended for the angular movement of the jaw (70) located at the top according to the figure.

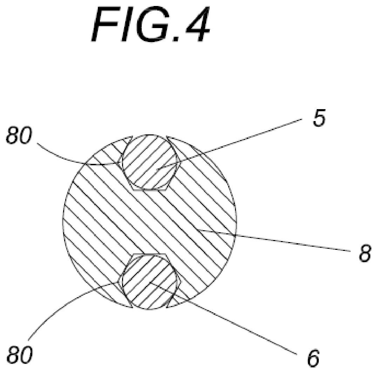

In order to protect the push rods (5, 6) from the external environment and from shocks, a cylindrical cover (8) (see Figure 1) is provided with a pair of longitudinal grooves (80) running parallel to each other, which are enabled for the arrangement of the push rods (5, 6), the cross-section of each of the grooves is a polygonal shape, for example, hexagonal as can be seen in Figure 4. This ensures that in the event of applying a great stress to the push rods (5, 6) caused by the jaws (70, 71), they are prevented from deforming and thus rendering the instrument useless (1).

Particular reference to the head (7) comprises the two movable jaws (70, 71) hinged to each other through a pivoting shaft (72), and an actuator system connected to each of the jaws in such a way that they are capable of moving rotatively independently, the actuator system being enabled to connect to movable gripping members (2, 3).

The actuator system comprises an actuator set for each of the jaws (70, 71) which has a pusher member (73) enabled to be coupled to pushrods (5, 6, 10) through a transmitter assembly (11), indicated in general with the reference (11), each pusher member (73) being linked in rotary motion with a corresponding jaw (70, 71) via a connecting rod member (74). In this way, each of the jaws (70, 71) is able to rotating with respect to the connecting rod member (74) when the pusher member (73) is displaced, in turn caused by the action of the movable gripping members (2, 3).

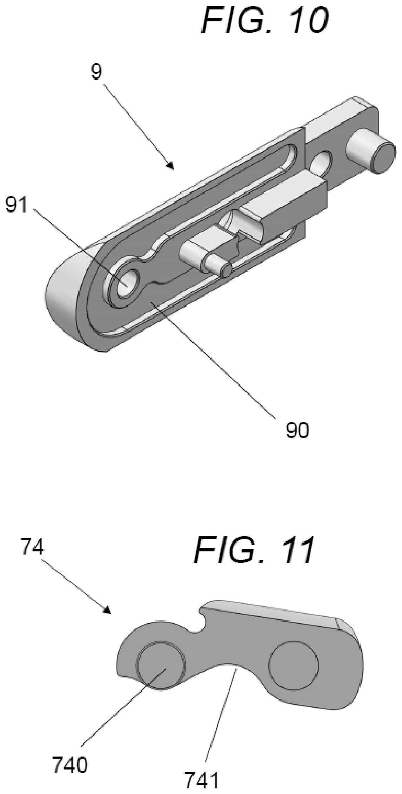

As can be seen in Figure 11, the connecting rod member (74) has an oblong development provided on one side of a curved contour recess (741) intended to accommodate transversely the pivoting shaft (72).

There is also the possibility that the two jaws (70, 71) can also rotate in an axis transverse to the longitudinal axis of the head, i.e. to the left or right, in such a way that they can rotate 90° to each of the two sides.

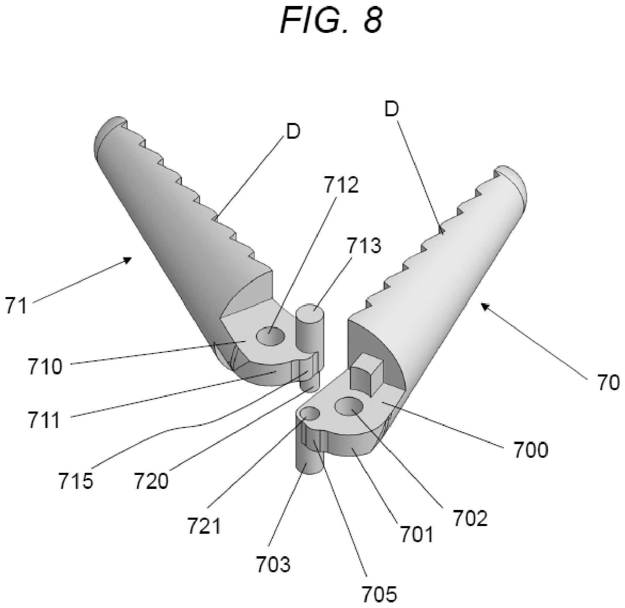

In this exemplary embodiment, each of the jaws (70, 71) is made up of an oblong metal body that has an edge with a serrated region, indicated by reference (D) in Figure 8, designed to grip and cut fabrics and a rear region (700, 710) provided with two flat seat sections in which the connecting rod member (74) is partially located.

As can be seen in Figure 8, the pivoting shaft (72) of the two jaws (70, 71) is made up of a protruding portion (720) that extends internally from one of the jaws and can be rotatingly coupled to a recess (721) present on the inside of the opposite jaw. This protruding portion (720) is positioned with respect to the rear region (710), so that a section of it extends from a flange section (711), so that the pivoting shaft (72) is displaced with respect to the center of the rear region (700, 710).

It should be noted that during the angular movement made by each of the jaws (70, 71) there is a coupling relationship, such that a tangential arrangement is adopted between the flange section (701, 711) of the jaw (70, 71) located in the rear region (710, 700) and a flange section (730) of curved shape of the pusher member (73). In other words, the movement of each of the jaws (70, 71) is carried out by the pushing force exerted by the pusher member (73). Thus, it is possible to have the two jaws open up to 180 degrees to each other at any and, at the same time, this degree of opening regardless of the angular arrangement of the jaws (70, 71) with respect to the longitudinal axis of the laparoscopic surgical instrument (1).

Therefore, the forward movement (i.e. the action of separation or closing) of each of the jaws (70, 71) is carried out by the pushing force exerted by the pusher member (73) in the flange sections (701, 711), and additionally with the connecting rods (74), while the movement in the action of retracting or opening the jaws (70, 71) is only transmitted by the connecting rod member (74).

This configuration makes it possible to reinforce the rigidity of the head at times when a great force is required, for example, during a surgical operation in which the closing position of the two jaws is required to carry out a cut of a very hard tissue, such as cartilage.

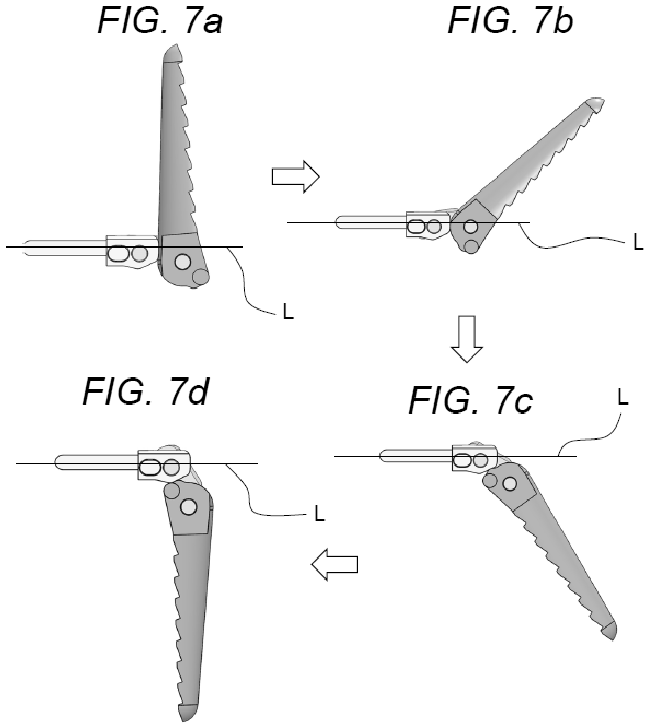

In figures 7a-d this tangential coupling relationship can be seen in a sequence of movement, where figure 7a shows a jaw positioned 90° with respect to the longitudinal axis (L) of the pusher member (73); in Figure 7b the jaw is located 45° with respect to the longitudinal axis (L) of the pusher member (73); in Figure 7c the jaw is located -45° with respect to the longitudinal axis (L) of the pusher member (73) and; in Figure 7d the jaw is located -90° to the longitudinal axis (L) of the pusher member (73). As can be seen, during the entire stroke of each jaw, there is a tangency relationship, i.e. at least one point of contact between the jaw (70, 71) and the pusher member (73) during the rotation of the jaw (70, 71), so that the pusher member (73) transforms the linear pushing action into a rotational movement of the jaw.

Thus, as shown from figures 7a-d, a cam system is provided between the jaw and the pusher member, such a system being formed by at least one protrusion (732) present in the flange section (730) of the pusher member and a protrusion (705, 715) in the flange section (701, 711) of the rear region (710, 700).

The connecting rod member (74) of each of the actuator sets is coupled in a hinged manner to its respective jaw by means of a lug (740) that is coupled in a through hole (702, 712) drilled in the rear region (700, 710) of each of the jaws (70, 71), as can be seen more clearly in Figure 8.

In addition, guide means are provided to guide the path of the connecting rod member (74) and the pusher member (73) connected to a jaw (70, 71) during the angular movement of the jaw.

For each of the jaws (70, 71), such guide means include a projection (731) protruding from a side face of the pusher member (73) which is movable along a "C" shaped slot (90) drilled into a cover (9), and a protrusion protruding from a lateral face of the connecting rod member (74) which is movable along the length of the slot (90) present in the cover (9), as can be seen in Figure 10. Each of the caps (9) is attached to a corresponding jaw (70, 71) by means of a lug (703, 713) present in each of the caps (70, 71) which is coupled to a through hole (91) of the lid (9). Thanks to the fact that the connecting rod member (74) and the lug (740) enter the slot (90) supporting each other throughout the stroke of the movement, the breakage of the protruding portion (720) can be avoided.

The details, shapes, dimensions and other accessory elements used in the manufacture of the head for a surgical instrument of the invention may be conveniently replaced by others which do not depart from the scope defined by the claims set out below.

## Claims

1. A head for a laparoscopic surgical instrument, comprising two jaws (70, 71) hinged together through a pivoting shaft, and an actuator system connected to each of the jaws (70, 71) such that they are able to move rotationally independently, the actuator system being enabled to connect to movable gripping members,
the actuator system comprises an actuator set for each of the jaws (70, 71) which has a pusher member (73) enabled to be coupled to a pushrod (5, 6), the pusher member (73) being linked to a corresponding jaw through a connecting rod member (74), in such a way that the jaw (70, 71) is capable of rotating with respect to the connecting rod member (74) by a linear displacement of the pusher member (73),
in which the pivoting shaft (72) that joins the two jaws (70, 71) is constituted by a tongue and groove system formed by two parts (720, 721) aligned with each other, and
wherein a tangential arrangement is adopted between a section of the jaw edge (70, 71) and a section of the edge of the pusher member (73) during the full stroke of the jaw rotation (70, 71),
each of the jaws (70, 71) comprises a body with an oblong development formed by a toothed region and a rear region (710, 700), the region (710, 700) having a flange section (701, 711) with a curved shape and,
the pusher member (73) comprising a flange section (730) enabled to have at least one point of contact with the flange section (701, 711) of the rear region (710, 700),
**characterized in that** a cam system is provided between the jaw and the pusher member, such a system being formed by at least one protrusion (732) present in the flange section (730) of the pusher member (73) and at least one protrusion (705, 715) in the flange section (701, 711) of the rear region (710, 700).

2. The head according to claim 1, **characterised in that** the connecting rod member (74) has an oblong development provided on a longer side of a curved contour recess intended to accommodate transversely the pivoting shaft (72).

3. The head according to claim 1, **characterised in that** the hinged connection between the connecting rod member (74) of each of the actuator sets and their respective jaw is by means of the arrangement of lugs (740) coupled in through holes drilled in the rear region (700, 710) of each of the jaws (70, 71).

4. The head according to claim 1, **characterised in that** guide means are provided to guide the path of the connecting rod member (74) and the pusher member connected to a jaw during the angular movement of the jaw (70, 71).

5. The head according to claim 4, **characterised in that** the guide means comprise at least one projection protruding from a side face of the pusher member (73) which is movable along a slot present in a cover (9).

6. The head according to claim 4, **characterised in that** the guide means comprise at least one protrusion protruding from a side face of the connecting rod member which is movable along a slot present in a cover (9).

7. The head according to any of claims 5 to 6, **characterized in that** each actuator set comprises a cap (9).

8. The head according to claim 1, **characterised in that** the tongue and groove system of the pivoting shaft (72) consists of a protruding portion (720) extending internally from one of the jaws (71) which is able to rotately attach to a recess (721) present in the opposite jaw (70).

9. The head according to claim 1, **characterised in that** the pivoting shaft hinging the two jaws (70, 71) is located at a point proximal to the flange present in the rear region (700, 710) of the jaw.

10. Laparoscopic surgical instrument comprising:
A hand-gripping region provided with two movable gripping members hinged to perform an angular and independent movement, each enabled to insert a user's finger, and a third fixed gripping member enabled to insert a user's finger, and
An actuator mechanism provided with push rods which are each coupled to a corresponding movable gripping member in such a way as to transmit the angular movement of the gripping members to a head according to claims 1 to 9.

11. Laparoscopic surgical instrument according to claim 10, **characterised in that** it includes a cylindrical cover provided with at least a pair of longitudinal grooves which are enabled for the arrangement of the push rods, the cross-section of the grooves is a polygonal shape.
